**Europäisches Patentamt**

**European Patent Office**

**Office européen des brevets**

(11) Veröffentlichungsnummer: **0 025 889**
**B1**

# EUROPÄISCHE PATENTSCHRIFT

(45) Veröffentlichungstag der Patentschrift:
**30.11.83**

(51) Int. Cl.³: **G 01 N 33/72, C 07 D 231/38**

(21) Anmeldenummer: **80105112.9**

(22) Anmeldetag: **28.08.80**

(54) **Diagnostisches Mittel zum Nachweis von Bilirubin in Körperflüssigkeiten und Verwendung eines dafür geeigneten Reagenzes.**

(30) Priorität: **12.09.79 DE 2936745**

(43) Veröffentlichungstag der Anmeldung:
**01.04.81 Patentblatt 81/13**

(45) Bekanntmachung des Hinweises auf die Patenterteilung:
**30.11.83 Patentblatt 83/48**

(84) Benannte Vertragsstaaten:
**AT BE CH DE FR GB IT LI LU NL SE**

(56) Entgegenhaltungen:
**DE - A - 2 229 611**
**DE - A - 2 521 402**
**DE - A - 2 623 087**

**Die Akte enthält technische Angaben, die nach dem Eingang der Anmeldung eingereicht wurden und die nicht in dieser Patentschrift enthalten sind.**

(73) Patentinhaber: **BEHRINGWERKE AKTIENGESELLSCHAFT, Postfach 1140, D-3550 Marburg/Lahn (DE)**

(72) Erfinder: **Kohl, Helmut, Dr., Goethestrasse 32, D-3552 Wetter (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

## Diagnostisches Mittel zum Nachweis von Bilirubin in Körperflüssigkeiten und Verwendung eines dafür geeigneten Reagenzes

Die Erfindung betrifft ein diagnostisches Mittel zum Nachweis von Bilirubin in Körperflüssigkeiten, enthaltend ein Diazoniumsalz.

Die analytische Bestimmung von Bilirubin mit Diazoniumsalzen ist eine gebräuchliche Methode in der klinischen Diagnostik von Leber- und Gallenerkrankungen. Beispiele hierfür sind in DE-C-2 240 471, DE-A-2 007 013, DE-A-2 364 844, DE-A-2 013 558, CH-512 072 beschrieben. Zur Herstellung der diagnostischen Mittel wird in der Regel ein hochreines Diazoniumsalz — z. B. ein Halogenbenzoldiazoniumsalz —, gelöst in Wasser, ohne weitere Zuschläge, in einem gut verschließbaren Behälter lyophilisiert und dort auch aufbewahrt. Bei Lagertemperaturen um 0°C unter Ausschluß von Licht und Luftfeuchtigkeit sind die Diazoniumsalze dann ausreichend lagerstabil.

Auf Schnelldiagnostika wie z. B. Teststreifen hingegen kann man diese idealen Bedingungen nicht übertragen. Hier würden sich viele der bisher verwendeten Diazoniumsalze sehr schnell zu dunkel gefärbten Folgeprodukten zersetzen. Die Stabilität der Diazoniumsalze ist aber für den diagnostischen Wert solcher Schnelldiagnostika, vor allem Testpapieren, von entscheidender Bedeutung. Es zeigt sich nämlich, daß schon schwach verfärbte Papiere bei Gebrauch zum Bilirubinnachweis im Urin im entscheidenden physiologisch-pathologischen Bereich zwischen 0,5 mg/dl und 2 mg/dl Bilirubin zu Fehlinterpretationen Anlaß geben. Diese Verfärbungen treten verstärkt in Gegenwart von Akzeleratoren in solchen Diagnostika auf. Letztere werden im allgemeinen benötigt, um einen ausreichend schnellen und empfindlichen Nachweis des Bilirubins zu ermöglichen. Parallel zur Zunahme dieser Verfärbungen nimmt während der Lagerung der Diagnostika der Gehalt pro cm² an Diazoniumsalzen stark ab, so daß auch von dieser Seite der Gebrauchswert bzw. die Laufzeit von Testpapieren zur Bilirubinbestimmung stark begrenzt wird.

Testpapiere werden daher unter Verwendung von die Diazoniumsalze stabilisierenden Zuschlägen hergestellt. Dieses sind z. B. die Aryl-Sulfonsäuren oder auch meta-Phosphorsäure. Trotz dieser Zuschläge sind die Testpapiere selten länger als 24 Monate verwendbar.

Solche Diazoniumsalze enthaltenden Diagnostika sind in den deutschen Offenlegungsschriften 2 623 087, 2 521 402 und 2 229 611 beschrieben. Ein nach D-A-2 623 087 hergestelltes Testpapier ist jedoch nicht stabil und kann unspezifisch mit anderen als dem nachzuweisenden Harninhaltsstoff reagieren. Das in D-A-2 521 402 beanspruchte Diazoniumsalz reagiert zwar mit Urobilinogen, jedoch nicht mit Bilirubin. Auch in D-A-2 229 611 sind Diazoniumsalze für den Nachweis von Urobilinogen beschrieben, die nur in Ausnahmefällen mit Bilirubin reagieren.

Ziel der vorliegenden Erfindung war daher, die aufgezeigten Mängel der diagnostischen Mittel zum Nachweis von Bilirubin, vor allem den Mangel der verwendeten Diazoniumsalze, zu beheben. Es sollte ein Diazoniumsalz Verwendung finden, welches sich bei der Lagerung nicht zersetzt bzw. nicht zu gefärbten Abbauprodukten zerfällt.

Die gestellte Aufgabe wird dadurch gelöst, daß als Indikator für Bilirubin ein Diazoniumsalz des Pyrazols eingesetzt wird.

Gegenstand der Erfindung ist die Verwendung eines Salzes des 3-Diazopyrazols als Reagenz für Bilirubin. Bevorzugt wird 3-Diazopyrazol-Tetrafluoroborat. Gegenstand der Erfindung ist vor allem ein diagnostisches Mittel zum Nachweis von Bilirubin in Körperflüssigkeiten, insbesondere im Urin, bestehend aus einem saugfähigen Träger, vor allem Papier, der mit einer nicht flüchtigen Säure, einem Netzmittel und einem Diazoniumsalz imprägniert ist, dadurch gekennzeichnet, daß das Diazoniumsalz ein 3-Diazopyrazolsalz ist. Bevorzugt wird das Tetrafluoroborat.

Weitere geeignete Reagenzien sind andere Salze des Diazopyrazols, z. B. Trifluoromethylsulfonate, Arylsulfonate, Chloride, Sulfate, Hexachlorantimonate, Tetrachlorzinkate des Diazopyrazols.

Für die Herstellung von Teststreifen als Schnelldiagnostika für Bilirubin nimmt man zweckmäßig als saugfähigen Träger Papier, welches mit einer Lösung imprägniert wird, die enthält 0,01 bis 0,4 M/l des erfindungsgemäßen Reagenzes, ggf. 0,1 bis 3 M/l einer nicht flüchtigen Säure, z. B. m-Phosphorsäure, einen Akzelerator, das sind Netzmittel, wie z. B. N-Ethyl-N,N-dimethyl-N-dodecyl-ammoniumbromid, der in der Tränklösung in einer Konzentration von 0,1%—7% vorliegt. Bevorzugt ist eine Konzentration von 0,5%—3%. In denselben Konzentrationen können aber auch andere Netzmittel, anionische, nichtionische oder kationische verwendet werden.

Bei vergleichenden Untersuchungen mit den Reagenzien des Standes der Technik zeigt sich überraschend der unerwartet große Vorteil der Erfindung.

In einem Vergleichsversuch wurden Testpapiere hergestellt

1) mit 2,4-Dichlorbenzoldiazonium-Tetrafluoroborat (Stand der Technik) und
2) mit Pyrazol-3-diazonium-Tetrafluoroborat (Erfindung).

Die Herstellung der Diazoniumsalze kann nach Angaben gemacht in WEYGAND-HILGETAG »Organisch-chemische Experimentierkunst«, 3. Aufl., J. A. Barth-Verlag, Leipzig, 1966, S. 641—643, erfolgen.

Die Papiere enthielten keine stabilisierende Sulfonsäure. Schon nach 10 Tagen Lagerung bei 4°C war das Papier 1) hellbraun gefärbt, bei einer 50°C-Lagerung wurde das Papier 1) dunkelbraun. Unter den gleichen Lagerbedingungen blieb das Papier 2) dagegen weiß. Farbabstufungen waren im Gebrauchstest mit dem Papier 2) auch bei geringen Bilirubin-Konzentrationen noch sehr gut wahrnehmbar. Mit dem Papier 1) konnten hingegen nur noch hohe Bilirubinkonzentrationen festgestellt werden. Der Diazoniumsalzgehalt hatte beim Papier 1) während der Versuchszeit bei 50°C um 60% abgenommen, beim Papier 2) war keine Konzentrationsveränderung meßbar.

In einem weiteren Versuch wurden Papiere zur Bilirubinbestimmung ohne die Verwendung von meta-Phosphorsäure und von Sulfonsäure hergestellt. Das Papier 2) enthielt wieder das erfindungsgemäße Pyrazol-3-diazonium-Tetrafluoroborat, das Papier 1) 2,4-Dichlorbenzol-diazonium-Tetrafluoroborat. Nach 10 Tagen Lagerung bei 50°C war das Papier 1) dunkelbraun gefärbt und reagierte nicht mehr mit Bilirubin. Mit dem Papier 2) erhielt man gelbliche Papiere, die noch zur differenzierenden Bilirubinbestimmung verwendbar waren. Auf dem Papier 1) konnte man kein Diazoniumsalz mehr nachweisen, auf dem Papier 2) hatte dessen Gehalt nur um 10% abgenommen.

Testpapiere mit Pyrazol-3-diazoniumsalz und einem quartären Ammoniumsalz als Akzelerator zeigen im Gebrauchstest einen weiteren Vorteil gegenüber den bisher üblichen Papieren: Das neue Diazoniumsalz reagiert mit Harnindikanen nur sehr langsam. Der Testbezirk bleibt in einem Bilirubin-freien Urin nahezu farblos, bei Verwendung von halogenierten Diazoniumsalzen verfärben sich die Testbezirke gelb. Farbveränderungen sind bei dieser Untergrundfarbe aber nur schwer wahrnehmbar.

Das folgende Beispiel erläutert die Erfindung:

Beispiel

1. Herstellung von Pyrazol-3-diazonium-Tetrafluoroborat

| | |
|---|---|
| 1 Mol | 3-Aminopyrazol werden vermischt mit |
| 1 l | Diethylether. Unter Rühren werden nacheinander vorsichtig zugetropft |
| 1 Mol | Fluoroborsäure und |
| 1,1 Mol | Isopentylnitrit, gelöst in |
| 1 l | Diethylether. |

Der ausfallende Niederschlag ist das Pyrazol-3-diazonium-Tetrafluoroborat.

2. Herstellung eines Bilirubin-Testpapiers

In einem Liter Wasser werden gelöst

| | |
|---|---|
| 10 g | m-Phosphorsäure |
| 50 g | Citronensäure |
| 30 g | Dodecylbenzolsulfat |
| 20 g | Pyrazol-3-diazoniumtetrafluoroborat |

Papier Schleicher und Schüll 2316 wird mit der Tränklösung imprägniert. Das bei 50°C getrocknete Papier ist weiß und zeigt die beschriebenen Effekte.

Bei Bedarf können dem Ansatz noch bis zu 100 g Naphthalin-1,5-disulfonsäure bzw. ein weiterer Stabilisator beigegeben werden.

Statt des obengenannten Netzmittels Dodecylbenzolsulfat kann auch ein anderes anionisches, aber auch ein kationisches Netzmittel wie z. B. N-Ethyl-N,N-dimethyl-N-dodecyl-ammoniumbromid eingesetzt werden.

**Patentansprüche**

1. Diagnostisches Mittel zum Nachweis von Bilirubin in Körperflüssigkeiten, bestehend aus einem saugfähigen Träger, der mit einer nicht flüchtigen Säure, einem Netzmittel und einem Diazoniumsalz imprägniert ist, dadurch gekennzeichnet, daß das Diazoniumsalz ein Salz des 3-Diazopyrazols ist.

2. Verwendung eines Salzes des 3-Diazopyrazols als Reagenz für Bilirubin.

**Claims**

1. A diagnostic agent for the determination of bilirubin in body fluids, consisting of an absorbent carrier which is impregnated with a non-volatile acid, a wetting agent and a diazonium salt, wherein

3

the diazonium salt is a salt of diazopyrazole.

2. Method of using a salt of 3-diazopyrazole as reagent for bilirubin.

**Revendications**

1. Agent de diagnostic pour la détection de la bilirubine dans des liquides physiologiques, constitué d'un support absorbant, qui est imprégné d'un acide non volatil, d'un agent mouillant et d'un sel de diazonium, caractérisé en ce que le sel de diazonium est un sel du 3-diazopyrazole.

2. Utilisation d'un sel du 3-diazopyrazole comme réactif pour la bilirubine.